# EUROPEAN PATENT APPLICATION

(11) **EP 2 708 238 A1**
(43) Date of publication of application: **19.03.2014**
(21) Application number: 12786584.8
(22) Date of filing: 24.04.2012
(51) Int. Cl.: A61K 39/00, A61K 35/76, A61K 39/12, A61K 39/145, A61P 31/12, A61P 35/00, C07K 14/025, C12N 15/09

(54) **CYTOTOXIC T CELL INDUCER**

(30) Priority: 13.05.2011 JP 2011107874; 02.09.2011 JP 2011191208
(71) Applicant: Tokyo Institute of Technology, Tokyo 152-8550 (JP); Saitama Medical University, Iruma-gun, Saitama 350-0495 (JP)
(72) Inventor: HANDA, Hiroshi, Tokyo 152-8550 (JP); KAWANO, Masaaki, Iruma-gun Saitama 350-0495 (JP); MATSUI, Masanori, Iruma-gun Saitama 350-0495 (JP)
(74) Representative: Cabinet Plasseraud
(86) International application number: PCT/JP2012/060910
(87) International publication number: WO 2012/157408

(57) **Abstract**

The purpose of the present invention is to allow a peptide to exhibit a sufficient effect as a vaccine in the absence of any adjuvant. Provided is a cytotoxic T cell inducer comprising virus-like particles each of which is composed of VP1 of simian virus 40 and which is characterized in that a T cell epitope peptide is inserted in a DE loop and/or an HI loop of the VP1.

SEQUENCE LISTING

<110> Tokyo Institute of Technology
      Saitama Medical University

<120> CYTOTOXIC T LYMPHOCYTE INDUCER

<130> FP-165PCT

<150> JP 2011/107874
<151> 2011-5-13

<150> JP 2011/191208
<151> 2011-9-2

<160> 52

<170> PatentIn Ver. 2.1

<210> 1
<211> 9
<212> PRT
<213> Influenzavirus

<400> 1
Gly Ile Leu Gly Phe Val Phe Thr Leu
  1               5


<210> 2
<211> 8
<212> PRT
<213> Influenzavirus

<400> 2
Ala Ser Cys Met Gly Leu Ile Tyr
  1               5


<210> 3
<211> 9
<212> PRT
<213> Influenzavirus

<400> 3
Ser Ile Ile Pro Ser Gly Pro Leu Lys
  1               5


<210> 4
<211> 10
<212> PRT
<213> Influenzavirus

<400> 4
Ile Leu Gly Phe Val Phe Thr Leu Thr Val
  1               5                  10


<210> 5
<211> 9
<212> PRT

<213> Influenzavirus

<400> 5
Cys Thr Glu Leu Lys Leu Ser Asp Tyr
1               5

<210> 6
<211> 9
<212> PRT
<213> Influenzavirus

<400> 6
Leu Pro Phe Glu Lys Ser Thr Val Met
1               5

<210> 7
<211> 9
<212> PRT
<213> Influenzavirus

<400> 7
Ile Leu Arg Gly Ser Val Ala His Lys
1               5

<210> 8
<211> 9
<212> PRT
<213> Influenzavirus

<400> 8
Glu Leu Arg Ser Arg Tyr Trp Ala Ile
1               5

<210> 9
<211> 9
<212> PRT
<213> Influenzavirus

<400> 9
Ser Arg Tyr Trp Ala Ile Arg Thr Arg
1               5

<210> 10
<211> 9
<212> PRT
<213> Influenzavirus

<400> 10
Asn Met Leu Ser Thr Val Leu Gly Val
1               5

<210> 11
<211> 10
<212> PRT
<213> Influenzavirus

<400> 11

Arg Ile Phe Leu Ala Met Ile Thr Tyr Ile
1               5                   10

<210> 12
<211> 9
<212> PRT
<213> Influenzavirus

<400> 12
Val Ser Asp Gly Gly Pro Asn Leu Tyr
1               5

<210> 13
<211> 9
<212> PRT
<213> Influenzavirus

<400> 13
Val Leu Thr Gly Asn Leu Gln Thr Leu
1               5

<210> 14
<211> 9
<212> PRT
<213> Influenzavirus

<400> 14
Ile Ile Leu Lys Ala Asn Phe Ser Val
1               5

<210> 15
<211> 9
<212> PRT
<213> Homo sapiens

<400> 15
Ala Leu Cys Arg Trp Gly Leu Leu Leu
1               5

<210> 16
<211> 9
<212> PRT
<213> Homo sapiens

<400> 16
Gln Leu Phe Glu Asp Asn Tyr Ala Leu
1               5

<210> 17
<211> 9
<212> PRT
<213> Homo sapiens

<400> 17
Lys Ile Phe Gly Ser Leu Ala Phe Leu
1               5

<210> 18
<211> 9
<212> PRT
<213> Homo sapiens

<400> 18
Ile Leu His Asn Gly Ala Tyr Ser Leu
1               5

<210> 19
<211> 9
<212> PRT
<213> Homo sapiens

<400> 19
Ile Ile Ser Ala Val Val Gly Ile Leu
1               5

<210> 20
<211> 9
<212> PRT
<213> Homo sapiens

<400> 20
Val Val Leu Gly Val Val Phe Gly Ile
1               5

<210> 21
<211> 9
<212> PRT
<213> Homo sapiens

<400> 21
Arg Leu Leu Gln Glu Thr Glu Leu Val
1               5

<210> 22
<211> 10
<212> PRT
<213> Homo sapiens

<400> 22
Val Met Ala Gly Val Gly Ser Pro Tyr Val
1               5                   10

<210> 23
<211> 9
<212> PRT
<213> Homo sapiens

<400> 23
Cys Leu Thr Ser Thr Val Gln Leu Val
1               5

<210> 24
<211> 9

<212> PRT
<213> Homo sapiens

<400> 24
Gln Leu Met Pro Tyr Gly Cys Leu Leu
1               5


<210> 25
<211> 8
<212> PRT
<213> Homo sapiens

<400> 25
Tyr Leu Glu Asp Val Arg Leu Val
1               5


<210> 26
<211> 9
<212> PRT
<213> Homo sapiens

<400> 26
Val Leu Val Lys Ser Pro Asn His Val
1               5


<210> 27
<211> 10
<212> PRT
<213> Homo sapiens

<400> 27
Tyr Met Ile Met Val Lys Cys Trp Met Ile
1               5                   10


<210> 28
<211> 9
<212> PRT
<213> Homo sapiens

<400> 28
Glu Leu Val Ser Glu Phe Ser Arg Met
1               5


<210> 29
<211> 9
<212> PRT
<213> Homo sapiens

<400> 29
Val Leu Arg Glu Asn Thr Ser Pro Lys
1               5


<210> 30
<211> 9
<212> PRT
<213> Homo sapiens

<400> 30
Arg Trp Gly Leu Leu Leu Ala Leu Leu
1               5

<210> 31
<211> 9
<212> PRT
<213> Homo sapiens

<400> 31
Thr Tyr Leu Pro Thr Asn Ala Ser Leu
1               5

<210> 32
<211> 9
<212> PRT
<213> Homo sapiens

<400> 32
Pro Tyr Val Ser Arg Leu Leu Gly Ile
1               5

<210> 33
<211> 9
<212> PRT
<213> Homo sapiens

<400> 33
Arg Met Phe Pro Asn Ala Pro Tyr Leu
1               5

<210> 34
<211> 9
<212> PRT
<213> Homo sapiens

<400> 34
Ser Leu Gly Glu Gln Gln Tyr Ser Val
1               5

<210> 35
<211> 9
<212> PRT
<213> Homo sapiens

<400> 35
Cys Met Thr Trp Asn Gln Met Asn Leu
1               5

<210> 36
<211> 9
<212> PRT
<213> Homo sapiens

<400> 36
Tyr Met Phe Pro Asn Ala Pro Tyr Leu
1               5

<210> 37
<211> 9
<212> PRT
<213> Homo sapiens

<400> 37
Cys Met Thr Trp Asn Gln Met Asn Leu
1               5


<210> 38
<211> 9
<212> PRT
<213> Homo sapiens

<400> 38
Cys Tyr Thr Trp Asn Gln Met Asn Leu
1               5


<210> 39
<211> 9
<212> PRT
<213> Homo sapiens

<400> 39
Arg Trp Pro Ser Cys Gln Lys Lys Phe
1               5


<210> 40
<211> 36
<212> DNA
<213> Artificial Sequence

<220>
<223> Description of Artificial Sequence:primer

<400> 40
aaaagtcgac accatgaaga tggccccaac aaaaag                                36


<210> 41
<211> 57
<212> DNA
<213> Artificial Sequence

<220>
<223> Description of Artificial Sequence:primer

<400> 41
cgtgaacaca aagcccaaaa tgccgccacc gccatgagtt ttttgtgtcc ctgaatg    57


<210> 42
<211> 56
<212> DNA
<213> Artificial Sequence

<220>
<223> Description of Artificial Sequence:primer

<400> 42
cattttgggc tttgtgttca cgttgggcgg cggtggtgct ggaaaaccca ttcaag     56


<210> 43
<211> 34
<212> DNA
<213> Artificial Sequence

<220>
<223> Description of Artificial Sequence:primer

<400> 43
aaaaggtacc tcactgcatt ctagttgtgg tttg                             34


<210> 44
<211> 36
<212> DNA
<213> Artificial Sequence

<220>
<223> Description of Artificial Sequence:primer

<400> 44
aaaagtcgac accatgaaga tggccccaac aaaaag                           36


<210> 45
<211> 34
<212> DNA
<213> Artificial Sequence

<220>
<223> Description of Artificial Sequence:primer

<400> 45
aaaaggtacc tcactgcatt ctagttgtgg tttg                             34


<210> 46
<211> 36
<212> DNA
<213> Artificial Sequence

<220>
<223> Description of Artificial Sequence:primer

<400> 46
aaaagtcgac accatgaaga tggccccaac aaaaag                           36


<210> 47
<211> 55
<212> DNA
<213> Artificial Sequence

<220>
<223> Description of Artificial Sequence:primer

<400> 47
cgtgaacaca aagcccaaaa tgccgccacc gccgttggta aacagcccac aaatg      55

<210> 48
<211> 54
<212> DNA
<213> Artificial Sequence

<220>
<223> Description of Artificial Sequence:primer

<400> 48
cattttgggc tttgtgttca cgttgggcgg cggtggaaca cagcagtgga aggg          54


<210> 49
<211> 34
<212> DNA
<213> Artificial Sequence

<220>
<223> Description of Artificial Sequence:primer

<400> 49
aaaaggtacc tcactgcatt ctagttgtgg tttg                                34


<210> 50
<211> 36
<212> DNA
<213> Artificial Sequence

<220>
<223> Description of Artificial Sequence:primer

<400> 50
aaaagtcgac accatgaaga tggccccaac aaaaag                              36


<210> 51
<211> 34
<212> DNA
<213> Artificial Sequence

<220>
<223> Description of Artificial Sequence:primer

<400> 51
aaaaggtacc tcactgcatt ctagttgtgg tttg                                34


<210> 52
<211> 20
<212> DNA
<213> Artificial Sequence

<220>
<223> Description of Artificial Sequence:synthetic DNA

<400> 52
tccatgacgt tcttgatgtt                                                20

## Description

### Technical Field

The present invention relates to a cytotoxic T lymphocyte (CTL) inducer, and a method for preventing or treating a viral disease or a cancer utilizing the same.

### Background Art

Simian virus 40 (SV40) is one of simian polyomaviruses belonging to Papovaviridae, and is a tumor virus which is small and has no envelope.

An outer shell of the SV40 virus is composed of a major capsid protein called VP1. When a VP1 gene is highly expressed in an insect cell or the like, VP1 is synthesized in a large amount, and is self-organized in a nucleus of a cell to form many virus-like particles (VLPs). It has been proposed by the present inventors that a drug or the like is encapsulated into this VLP, and the resultant is utilized as drug delivery (WO 2006/004173).

It has been confirmed by the present inventors that a normal VLP becomes not to be formed in many cases when an exogenous peptide is inserted into VP1, but normal particles are formed when an exogenous peptide is inserted into a DE loop or an HI loop in VP1. It is possible to insert an exogenous peptide into a DE loop or an HI loop utilizing this nature to change cell directionality of a VLP (Patent Document 1).

Preparation of a vaccine utilizing this VLP has previously been conducted. For example, Patent Document 2 describes a CTL vaccine containing a VLP with a CTL epitope peptide bound thereto.

### Prior Art Documents

### Patent Documents

Patent Document 1: WO 2006/088229
Patent Document 2: JP 2009-197001 A

### Disclosure of the Invention

### Problems to be Solved by the Invention

In order to destroy a virus-infected cell or the like, a virus-derived peptide is utilized as a vaccine. However, since a peptide is poor in retention and is easily degraded, it is usually administered with an adjuvant. However, many of adjuvants have some side effects, and there is a problem with administration to a human.

The present invention was made under the aforementioned technical background, and an object thereof is to provide a means which allows a peptide to exhibit a sufficient effect as a vaccine even in the absence of an adjuvant.

### Means for Solving the Problems

In order to solve the aforementioned problems, the present inventors intensively continued study and, as a result, found out that a VLP composed of altered VP1 in which a T cell epitope peptide is inserted into a DE loop or an HI loop exhibits the strong CTL inductivity.

A VLP composed of altered VP1 in which an exogenous peptide is inserted into a DE loop or an HI loop is disclosed in Patent Document 1. However, in Patent Document 1, an exogenous peptide is inserted exclusively for changing cell directionality of a VLP, and an idea of inducing a CTL using an exogenous peptide is not disclosed in Patent Document 1. In addition, in claim 10 or the like of Patent Document 1, use of an antigenic epitope as an exogenous peptide is described, but this antigenic epitope is used to enable a VLP to bind to an antibody. That is, the "epitope" described in Patent Document 1 is an epitope which is recognized by an antibody, but is not an epitope which is recognized by a T cell (T cell epitope).

A CTL vaccine containing a VLP with a CTL epitope peptide bound thereto is disclosed in Patent Document 2 (claim 182, etc.). However, Patent Document 2 does not disclose insertion of a CTL epitope peptide into a DE loop and/or an HI loop of VP1 of simian virus 40. The CTL inducer of the present invention has a sufficient CTL inducing effect even without an immunostimulator, but a vaccine disclosed in Cited Reference 2 contains an immunostimulator such as a non-methylated CpG-containing oligonucleotide (claim 1), and this point is also different from the CTL inducer of the present invention.

The present invention was completed based on the aforementioned findings.

That is, the present invention provides the following (1) to (10).
(1) A CTL inducer containing a virus-like particle composed of VP1 of simian virus 40, wherein a T cell epitope peptide is inserted into a DE loop and/or an HI loop of the VP1.
(2) The CTL inducer according to the item (1), wherein the T cell epitope peptide is a peptide derived from a virus protein.
(3) The CTL inducer according to the item (1), wherein the T cell epitope peptide is a peptide derived from HA, NA, M1, M2, NP, NS1, NS2, PA, PB1, PB2 or PB1-F2 of an influenza virus.
(4) The CTL inducer according to the item (1), wherein the T cell epitope peptide is a peptide derived from a protein specific to a cancer cell.
(5) The CTL inducer according to the item (1), wherein the T cell epitope peptide is a peptide derived from Melan-A/MART-1, gp100, MAGE-A3, MAGE-A10, CEA, HER2/new, NY-E50-1, WT-1 or hTERT.
(6) The CTL inducer according to the item (1), wherein the T cell epitope peptide is a peptide consisting of an amino acid sequence described in any of SEQ ID Nos.: 1 to 39.
(7) A method for preventing or treating a viral disease of an animal other than a human, including administering, to an animal other than a human, a virus-like particle composed of VP1 of simian virus 40 in which a T cell epitope peptide derived from a virus protein is inserted into a DE loop and/or an HI loop of the VP1.
(8) A method for preventing or treating a cancer of an animal other than a human, including administering, to an animal other than a human, a virus-like particle composed of VP1 of simian virus 40 in which a T cell epitope peptide derived from a protein specific to a cancer cell is inserted into a DE loop and/or an HI loop of the VP1.
(9) A method for preventing or treating a viral disease of a human, including administering, to a human, a virus-like particle composed of VP1 of simian virus 40 in which a T cell epitope peptide derived from a virus protein is inserted into a DE loop and/or an HI loop of the VP1.
(10) A method for preventing or treating a cancer of a human, including administering, to a human, a virus-like particle composed of VP1 of simian virus 40 in which a T cell epitope peptide derived from a protein specific to a cancer cell is inserted into a DE loop and/or an HI loop of the VP1.

### Effect of the Invention

The VLP contained in the CTL inducer of the present invention has the strong CTL inducing effect even without an adjuvant. Therefore, the CTL inducer of the present invention can prevent or treat a viral disease or a cancer without generating a side effect.

### Brief Description of Drawings

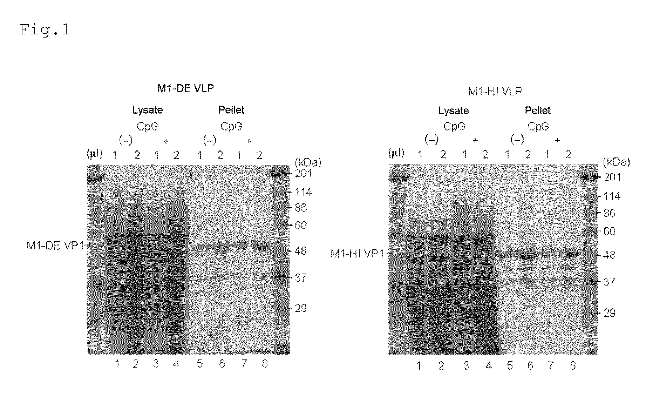

[Fig. 1] A view showing the result of CBB staining after SDS-PAGE of a lysate and a pellet solution of M1-DE-VLP and M1-HI-VLP. A part of the prepared lysate and pellet solution was developed on 10% SDS-PAGE, and stained with CBB. The lysate of M1-DE-VLP is shown in lanes 1 to 4 of the left view, and a sample not containing a CpG adjuvant was developed in lanes 1 and 2, and a sample containing a CpG adjuvant was developed in lanes 3 and 4. The pellet of M1-DE-VLP is shown in lanes 5 to 6 of the left view, and a sample not containing a CpG adjuvant was developed in lanes 5 and 6, and a sample containing a CpG adjuvant was developed in lanes 7 and 8. The lysate of M1-HI-VLP is shown in lanes 1 to 4 of the right view, and a sample not containing a CpG adjuvant was developed in lanes 1 and 2, and a sample containing a CpG adjuvant was developed in lanes 3 and 4. The pellet of M1-HI-VLP is shown in lanes 5 to 6 of the right view, and a sample not containing a CpG adjuvant was developed in lanes 5 and 6, and a sample containing a CpG adjuvant was developed in lanes 7 and 8.

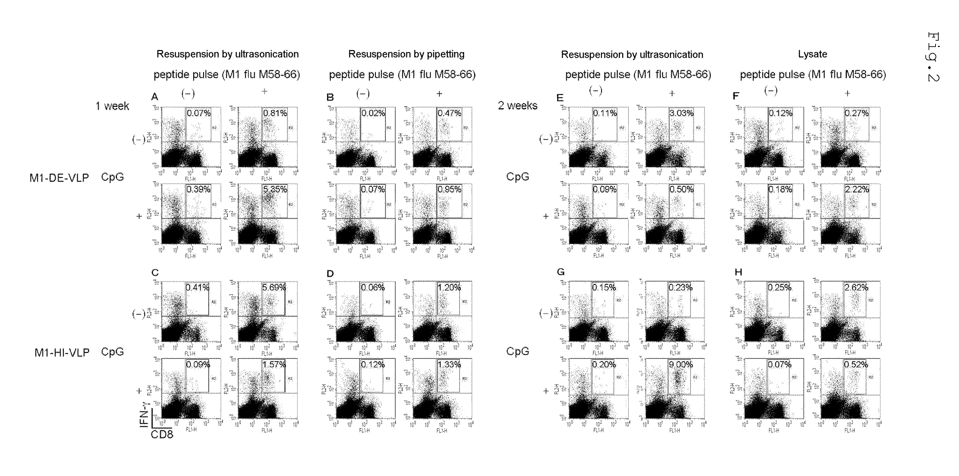

[Fig. 2] A view showing the analysis result of intracellular cytokine staining (ICS) after footpad immunization of a lysate and a pellet solution of M1-DE-VLP and M1-HI-VLP. A pellet solution of M1-DE-VLP (A, B) and M1-HI-VLP (C, D) (a sample was resuspended by ultrasonication (A, C) or resuspended by pipetting (B, D)) was immunized into a transgenic mouse, and this was analyzed with ICS after one week. Further, a pellet solution of M1-DE-VLP (E, F) and M1-HI-VLP (G, H) (a sample was resuspended by ultrasonication (E, G)) and a lysate solution of them (F, H) were immunized into a transgenic mouse, and this was analyzed with ICS after 2 weeks. CpG (-) indicates that a CpG adjuvant is not added, and CpG+ indicates that a CpG adjuvant is added. Further, peptide pulse (M1 flu M58-66) (-) indicates that a CTL epitope sequence (GILGFVFTL) is not added, and peptide pulse (M1 flu M58-66)+ indicates that a CTL is reinduced by adding the sequence. A dot in the right upper frame of each view represents a CD8 positive, intracellular IFN-γ positive cell, and the number in the frame indicates the ratio of this cell.

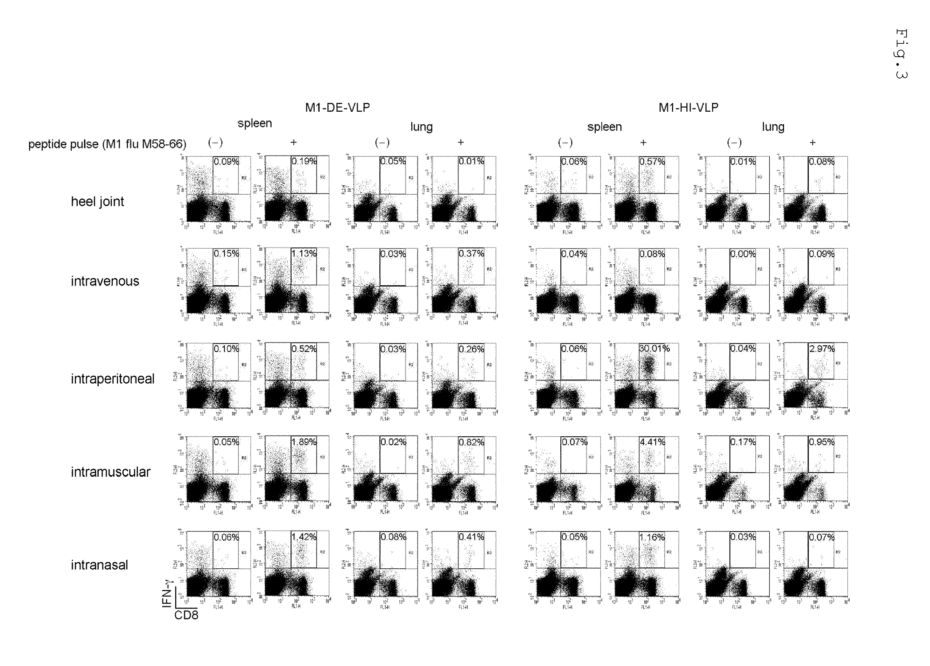

[Fig. 3] A view showing the ICS analysis result by different immune routes of M1-DE-VLP and M1-HI-VLP. A pellet solution of M1-DE-VLP (left view) and M1-HI-VLP (right view) (a sample was resuspended by ultrasonication) was immunized by a heel joint, intravenous, intraperitoneal, intramuscular or intranasal route, and ICS analysis was performed after one week. Peptide pulse (M1 flu M58-66) (-) indicates that a CTL epitope sequence (GILGFVFTL) is not added, and peptide pulse (M1 flu M58-66)+ indicates that a CTL is reinduced by adding the sequence. Further, spleen indicates lymphocytes extracted from the spleen, and lung indicates lymphocytes extracted from the lung. A dot in the right upper frame of each view represents a CD8 positive, intracellular IFN-γ positive cell, and the number in the frame indicates the ratio of this cell.

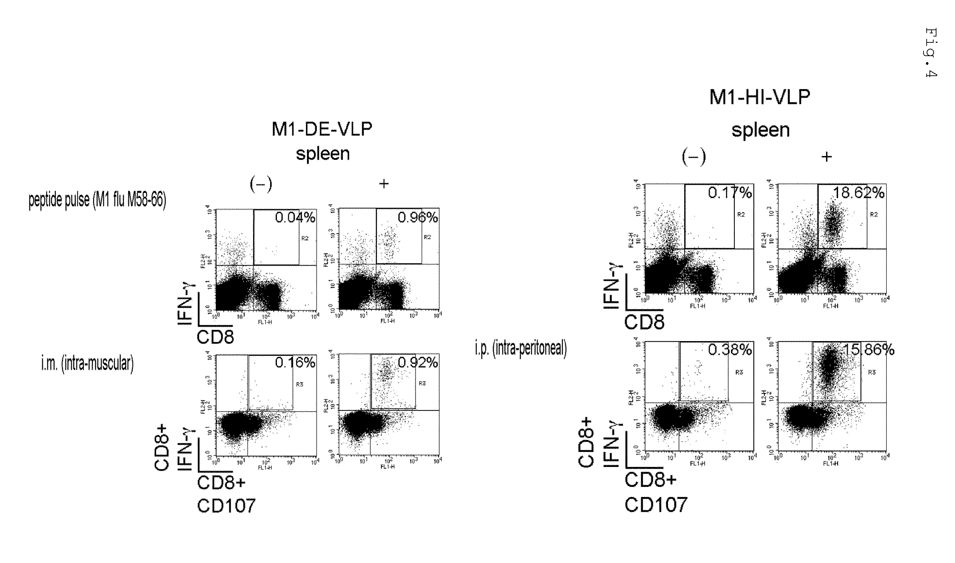

[Fig. 4] A view showing the CD107-ICS analysis result by immunization with M1-DE-VLP and M1-HI-VLP. A pellet solution of M1-DE-VLP (left view) and M1-HI-VLP (right view) (a sample was resuspended by ultrasonication) was immunized by an intramuscular or intraperitoneal route, and CD107-ICS analysis was performed after one week. Peptide pulse (M1 flu M58-66) (-) indicates that a CTL epitope sequence (GILGFVFTL) is not added, and peptide pulse (M1 flu M58-66)+ indicates that a CTL is reinduced by adding the sequence. Further, spleen indicates lymphocytes extracted from the spleen. A dot in the right upper frame of each view represents a CD8 positive, intracellular IFN-γ positive cell (upper view), and a CD107 positive, intracellular IFN-γ positive cell of a CD8 positive cell (lower view), respectively, and the number in the frame indicates the ratio of this cell.

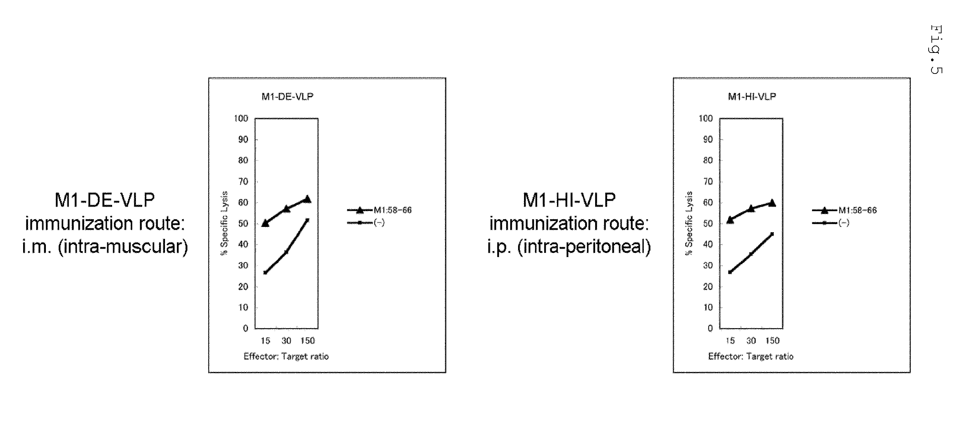

[Fig. 5] A view showing the ⁵¹Chrome release analysis result by immunization with M1-DE-VLP and M1-HI-VLP. A pellet solution of M1-DE-VLP (left view) and M1-HI-VLP (right view) (a sample was resuspended by ultrasonication) was immunized by an intramuscular or intraperitoneal route, lymphocytes were taken out after one week and cultured for ⁵¹Chrome release analysis, and ⁵¹Chrome release analysis was conducted after culturing for one week. (-) indicates the ratio of Chrome release from target cells with no CTL epitope sequence (GILGFVFTL) added thereto, and M1: 58-66 indicates the ratio of Chrome release from target cells with the sequence added thereto.

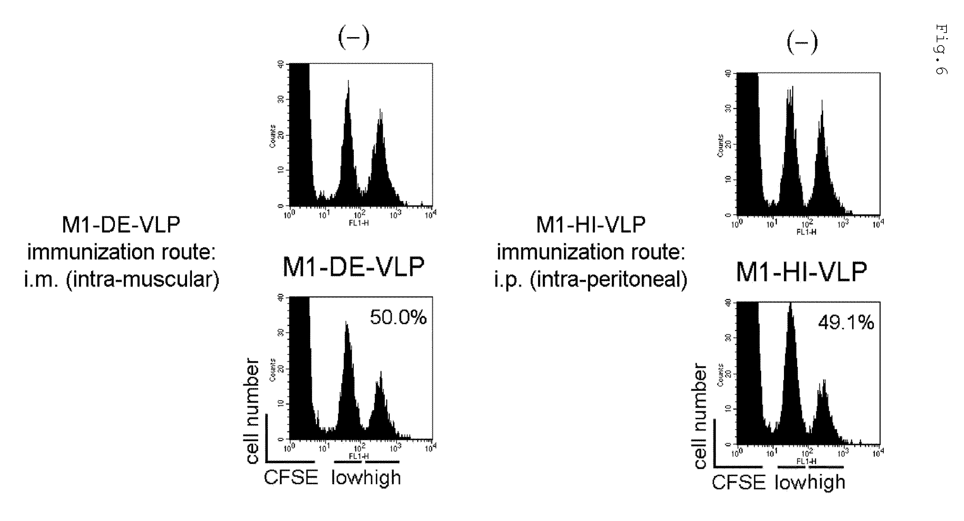

[Fig. 6] A view showing the In vivo CTL analysis result by immunization with M1-DE-VLP and M1-HI-VLP. A pellet solution of M1-DE-VLP (left view) and M1-HI-VLP (right view) (a sample was resuspended by ultrasonication) was immunized by an intramuscular or intraperitoneal route. After one week, same numbers of lymphocytes (CFSE high) with a CTL epitope sequence (GILGFVFTL) mixed therein and lymphocytes (CFSE low) with no such sequence mixed therein, which had been CFSE-stained, were introduced by intravenous injection and, further after one day, In vivo CTL analysis was performed. Each view indicates the CFSE intensity (abscissa) and the cell number (ordinate) of a CFSE-stained cell in a mouse lymphocyte. (-) in the graph indicates the case where lymphocytes of a non-immunized mouse were used, and M1-DE-VLP and M1-HI-VLP indicate the case where lymphocytes of mice immunized with M1-DE-VLP and M1-HI-VLP, respectively, were used. The number in the frame indicates the specific damage ratio on lymphocytes calculated by the following expression: specific damage ratio (%) = (1 - r(control)/r(immunized)) × 100, wherein r(control) represents (lymphocyte number of CFSE low/lymphocyte number of CFSE high) in a non-immunized mouse, and r(immunized) represents (lymphocyte number of CFSE low/lymphocyte number of CFSE high) in an immunized mouse.

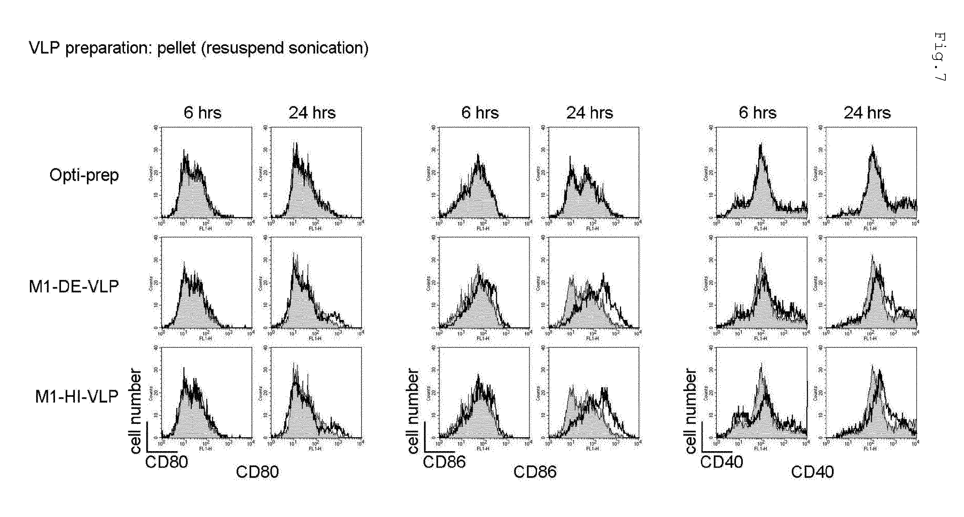

[Fig. 7] A view showing maturation of lymphocytes by addition of M1-DE-VLP and M1-HI-VLP. Lymphocytes with no substance added thereto (gray region in histogram), with a 50% Opti-prep™ solution added thereto (upper column, bold line in histogram, outlined), with M1-DE-VLP added thereto (middle column, bold line in histogram, outlined), or with M1-HI-VLP added thereto (lower column, bold line in histogram, outlined) were recovered after 6 hours (each left view) and after 24 hours (each right view), stained with a FITC-anti-CD80 antibody (left), FITC-anti-CD86 antibody (middle), or a FITC-anti-CD40 antibody (right), and subjected to histogram analysis. The ordinate indicates the cell number, and the abscissa indicates the intensity of FITC. In lymphocytes (bold line in histogram, outlined) which were incubated with M1-DE-VLP and M1-HI-VLP stained with a FITC-anti-CD86 antibody and a FITC-CD40 antibody, the histogram is biased right as compared with that of lymphocytes with no substance added thereto (gray region in histogram). This shows that a FITC-anti-CD86 antibody and a FITC-anti-CD40 antibody bound to a lymphocyte surface in a greater number as compared with lymphocytes with no substance added thereto, since CD86 and CD40 were expressed on a lymphocyte surface.

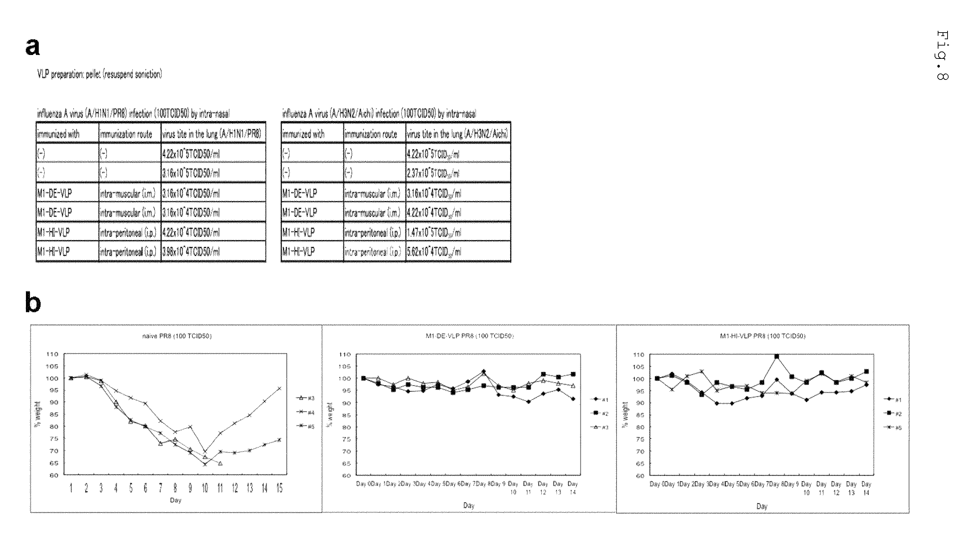

[Fig. 8] A view showing the analysis result of a virus challenge experiment on an influenza virus by immunization with M1-DE-VLP and M1-HI-VLP. [Fig. 8-a] A pellet solution of M1-DE-VLP and M1-HI-VLP (a sample was resuspended by ultrasonication) was immunized by an intramuscular or intraperitoneal route. After one week, an immunized mouse was anesthetized and nasally infected with an influenza A virus (A/H1N1/PR8 and A/H3N2/Aichi, concentration: 100 TCID₅₀/20 µl, inoculation amount: 20 µl), an extraction liquid was prepared from the lung after 4 days, and an amount of an influenza virus contained therein was calculated as a tissue culture influenza virus infectious dose (TCID₅₀) from the distribution of hemagglutination. The left table shows the result of the amount of an influenza virus of a mouse lung infected with A/H1N1/PR8, and the right table shows the result of the amount of an influenza virus of a mouse lung infected with A/H3N2/Aichi. Further, immunized with (-) indicates a tissue culture influenza virus infectious dose (TCID₅₀) of an amount of an influenza virus contained per 1 ml of an extraction liquid of the lung of a non-immunized mouse, M1-DE-VLP indicates the relevant amount of the lung of a mouse immunized with M1-DE-VLP, and M1-HI-VLP indicates the relevant amount of the lung of a mouse immunized with M1-HI-VLP. [Fig. 8-b] A pellet solution of M1-DE-VLP and M1-HI-VLP (a sample was resuspended by ultrasonication) was immunized by an intramuscular or intraperitoneal route. After one week, an immunized mouse was anesthetized and nasally infected with an influenza A virus (A/H1N1/PR8, concentration: 100 TCID₅₀/20 µl, inoculation amount: 20 µl), and a change in the weight of a mouse was analyzed every day for 2 weeks, regarding the weight of the mouse thereupon as 100%. The left view shows a change in the weight of a non-immunized mouse, the middle view indicates a change in the weight of a mouse immunized with M1-DE-VLP, and the right view indicates a change in the weight of a mouse immunized with M1-HI-VLP.

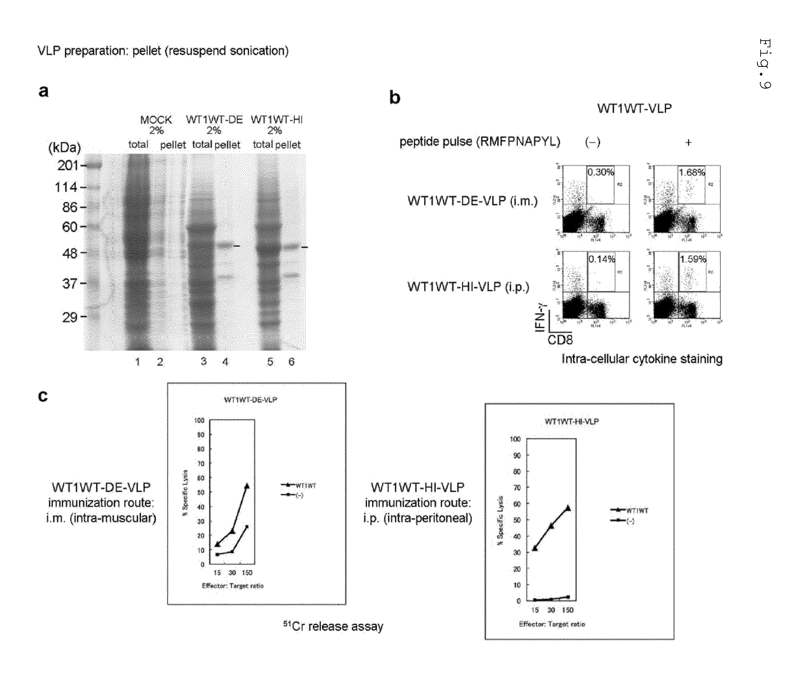

[Fig. 9] [Fig. 9-a] A view showing the result of CBB staining after SDS-PAGE of a lysate and a pellet solution of WT1WT-DE-VLP and WT1WT-HI-VLP. A part of the prepared lysate and pellet solution was developed on 10% SDS-PAGE, and stained with CBB. The lysate of WT1WT-DE-VLP is in the lane 3, the pellet of WT1WT-DE-VLP is in the lane 4, the lysate of WT1WT-HI-VLP is in the lane 5, and the pellet of WT1WT-HI-VLP is in the lane 6. [Fig. 9-b] A view showing the ICS analysis result in different immune routes of WT1WT-DE-VLP and WT1WT-HI-VLP. The pellet solution of WT1WT-DE-VLP (Fig. 9-b, upper column) and WT1WT-HI-VLP (Fig. 9-b, lower column) (a sample was resuspended by ultrasonication) was immunized by an intramuscular (WT1WT-DE-VLP) or intraperitoneal (WT1WT-HI-VLP) route, and ICS analysis was performed after one week. Peptide pulse (RMFPNAPYL) (-) indicates that a CTL epitope sequence (RMFPNAPYL) is not added, and peptide pulse (RMFPNAPYL)+ indicates that a CTL is reinduced by adding the sequence. A dot in the right upper frame of each view represents a CD8 positive, intracellular IFN-γ positive cell, and the number in the frame indicates the ratio of this cell. [Fig. 9-c] A view showing the ⁵¹Chrome release analysis result by immunization with WT1WT-DE-VLP and WT1WT-HI-VLP. A pellet solution of M1-DE-VLP (left view) and M1-HI-VLP (right view) (a sample was resuspended by ultrasonication) was immunized by an intramuscular or intraperitoneal route, lymphocytes were taken out after one week and cultured for ⁵¹Chrome release analysis, and ⁵¹Chrome release analysis was conducted after culturing for one week. (-) indicates the ratio of Chrome release from target cells with no CTL epitope sequence (RMFPNAPYL) added thereto, and WT1WT indicates the ratio of Chrome release from target cells with the sequence added thereto.

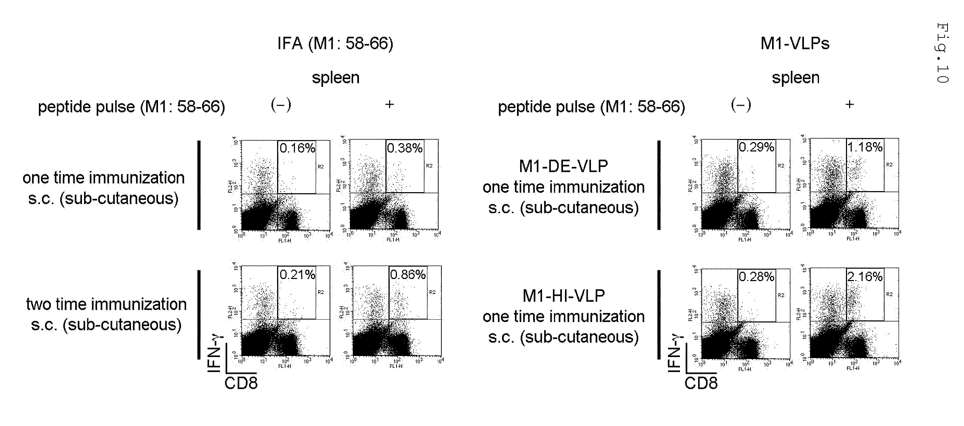

[Fig. 10] A view showing the result of comparison of CTL induction between IFA (incomplete freund's adjuvant) and M1-DE-VLP or M1-HI-VLP by ICS analysis. M1: ICS analysis was performed on a mouse after one week from the following two immunizations: first immunization (left upper view) with an emulsion obtained by suspending a peptide of a 58-66 CTL epitope sequence (GILGFVFTL) with IFA by a subcutaneous (s.c.) route, and second immunization (left lower view) one week after first immunization, and a mouse after one week from immunization with a pellet solution of M1-DE-VLP (right upper view) and M1-HI-VLP (right lower view) (a sample was resuspended by ultrasonication) by a subcutaneous (s.c.) route. Peptide pulse (M1: 58-66) (-) indicates that a CTL epitope sequence (GILGFVFTL) is not added, and peptide pulse (M1: 58-66)+ indicates that a CTL is reinduced by adding the sequence. Further, spleen indicates lymphocytes extracted from a spleen. A dot in the right upper frame of each view represents a CD8 positive, intracellular IFN-γ positive cell, and the number in the frame indicates the ratio of this cell.

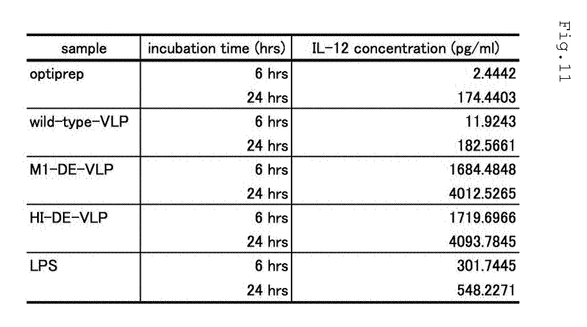

[Fig. 11] A view showing the result of ELISA analysis of an amount of secretion of IL-12 from a spleen-extracted lymphocyte when incubated with M1-DE-VLP and M1-HI-VLP. The optiprep in the left of the table indicates that incubation was performed with only a solvent in which a VLP is dissolved, wild-type-VLP indicates that incubation was performed with a wild-type VLP with no CTL epitope inserted therein, and LPS indicates that incubation was performed with lymphocytes extracted from the spleen with lipopolysaccharide. The middle column indicates the incubation time, and the right column indicates the concentration of IL-12 of the culturing supernatant of lymphocyte culturing, which was calculated by ELISA.

### Mode for Carrying Out the Invention

The present invention will be explained in detail below.

The CTL inducer of the present invention contains a virus-like particle composed of VP1 of simian virus 40, and is characterized in that a T cell epitope peptide is inserted into a DE loop and/or an HI loop of the VP1.

The T cell epitope peptide used in the present invention is not particularly limited, as far as it can induce a CTL. As the T cell epitope peptide, a peptide derived from a virus protein and a peptide derived from a protein specific to a cancer cell are known and, in the present invention, all of these peptides can be used. Herein, the "protein specific to a cancer cell" refers to a protein which is not expressed in a normal cell and is expressed only in a cancer cell, or a protein, expression of which is increased in a cancer cell as compared with that in a normal cell.

Examples of the peptide derived from a virus protein include peptides derived from proteins such as HA, NA, M1, M2, NP, NS1, NS2, PA, PB1, PB2 and PB1-F2 of an influenza virus. Among them, peptides derived from proteins such as M1, NP, NS1, PA, PB1 and PB2 of an influenza virus are preferable. Examples of specific peptides include GILGFVFTL (SEQ ID No.: 1), ASCMGLIY (SEQ ID No.: 2), SIIPSGPLK (SEQ ID No.: 3), and ILGFVFTLTV (SEQ ID No.: 4), which are peptides derived from M1 of an influenza virus, CTELKLSDY (SEQ ID No.: 5), LPFEKSTVM (SEQ ID No.: 6), ILRGSVAHK (SEQ ID No.: 7), ELRSRYWAI (SEQ ID No.: 8), and SRYWAIRTR (SEQ ID No.: 9), which are peptides derived from NP, NMLSTVLGV (SEQ ID No.: 10), RIFLAMITYI (SEQ ID No.: 11), and VSDGGPNLY (SEQ ID No.: 12), which are peptides derived from PB1, VLTGNLQTL (SEQ ID No.: 13), which is a peptide derived from PB2, and IILKANFSV (SEQ ID No.: 14), which is a peptide derived from NS1.

Examples of a peptide derived from a protein of a virus other than an influenza virus include peptides derived from Gag, Pol, Env, Tat, Nef and Rev of HIV, peptides derived from E1, E2, Core, NS2, NS3, NS4 and NS5 of a hepatitis C virus (HCV), and peptides derived from E6 and E7 of a human papilloma virus (HPV).

Examples of the peptide derived from a protein specific to a cancer cell include peptides derived from proteins such as Melan-A/MART-1, gp100, MAGE-A3, MAGE-A10, CEA, HER2/new, NY-E50-1, WT-1, and hTERT. Among them, peptides derived from proteins such as HER2/new, WT-1, and MAGE-A3 are preferable. Examples of specific peptides include ALCRWGLLL (SEQ ID No.: 15), QLFEDNYAL (SEQ ID No.: 16), KIFGSLAFL (SEQ ID No.: 17), ILHNGAYSL (SEQ ID No.: 18), IISAVVGIL (SEQ ID No.: 19), VVLGVVFGI (SEQ ID No.: 20), RLLQETELV (SEQ ID No.: 21), VMAGVGSPYV (SEQ ID No.: 22), CLTSTVQLV (SEQ ID No.: 23), QLMPYGCLL (SEQ ID No.: 24), YLEDVRLV (SEQ ID No.: 25), VLVKSPNHV (SEQ ID No.: 26), YMIMVKCWMI (SEQ ID No.: 27), ELVSEFSRM (SEQ ID No.: 28), VLRENTSPK (SEQ ID No.: 29), RWGLLLALL (SEQ ID No.: 30), TYLPTNASL (SEQ ID No.: 31), and PYVSRLLGI (SEQ ID No.: 32), which are peptides derived from HER2/new, and RMFPNAPYL (SEQ ID No.: 33), SLGEQQYSV (SEQ ID No.: 34), CMTWNQMNL (SEQ ID No.: 35), YMFPNAPYL (SEQ ID No.: 36), CMTWNQMNL (SEQ ID No.: 37), CYTWNQMNL (SEQ ID No.: 38), and RWPSCQKKF (SEQ ID No.: 39), which are peptides derived from WT-1.

The length of the T cell epitope peptide to be inserted into a loop is not limited as far as a CTL can be induced, but is usually 3 to 27 amino acids, preferably 8 to 11 amino acids, more preferably 9 amino acids.

The DE loop is located at the 127th to 146th positions counted from an N end of VP1, and the HI loop is located at the 268th to 277th positions (WO 2006/088229). The T cell epitope peptide may be inserted into any of these loops. As for the DE loop, it is preferable that the T cell epitope peptide is inserted so as to replace an amino acid sequence of the 137th to 138th positions with the epitope peptide. As for the HI loop, it is preferable that the T cell epitope peptide is inserted so as to replace an amino acid sequence of the 273rd to 274th positions with the epitope peptide. Into both ends of the T cell epitope peptide to be inserted into the loop, usually, 1 to several spacer amino acids, for example, glycine, alanine, and serine are inserted (WO 2006/088229). The number of spacer amino acids is usually 1 to 9, preferably 1 to 6, further preferably 3 to 6.

The T cell epitope peptide may be inserted into both of the DE loop and the HI loop, or may be inserted into only one of them. When it is inserted into both of them, the same T cell epitope peptide may be inserted, or different T cell epitope peptides may be inserted. Further, two or more T cell epitope peptides may be inserted into the loop in series.

Since a method for preparing the virus-like particle composed of VP1 in which an exogenous peptide is inserted into a loop is described in WO 2006/088229, a virus-like particle composed of VP1 in which the T cell epitope peptide is inserted into a loop can be prepared according to this method. For example, an objective virus-like particle can be prepared by preparing an altered VP1 gene in which a nucleotide sequence encoding the T cell epitope peptide is inserted, and highly expressing this altered VP1 gene in an insect cell or the like using a baculovirus vector or the like.

The CTL inducer of the present invention is formulated into a form of a general pharmaceutical composition in accordance with the nature of a substance as an active ingredient, and direct delivery of the composition can be generally attained by parenteral injection (e.g. subcutaneous injection, intraperitoneal injection, intravenous injection, intramuscular injection, or injection into an interstitial space of a tissue). Examples of other administration methods include mucous membrane administration (e.g. oral, transnasal, or lung), administration via eyes, transdermal administration and suppositories.

That is, when administered parenterally, the CTL inducer can be administered in a dosage form such as injectables, pernasal preparations, local preparations (transdermal preparations, etc.) and rectal preparations. When administered orally, the CTL inducer can be administered in a dosage form which is usually used in the art. Examples of the injectables include sterile solutions or suspensions and emulsions, specifically, water, a water-propylene glycol solution, a buffering solution, and 0.4% physiological saline. Further, when the CTL inducer is formulated into a liquid preparation, it can be freeze-preserved, or can be preserved after removing water by lyophilization or the like. Lyophilized preparations are used by adding distilled water for injection or the like and redissolved before use. Examples of the local preparations include creams, ointments, lotions, and transdermal preparations. Examples of the oral preparations or the rectal preparations include capsules, tablets, pills, powders, drops, suppositories, and solutions.

The aforementioned dosage forms are formulated into preparations together with pharmaceutically acceptable excipients and additives by a procedure which is usually performed in the art. Examples of the pharmaceutically acceptable excipients and additives include carriers, binders, perfumes, buffers, thickeners, colorants, stabilizers, emulsifiers, dispersants, suspending agents, antiseptics, pH adjusting agents, tonicity adjusting agents, and wetting-out agents. Further, examples of the pharmaceutically acceptable carriers include magnesium carbonate, lactose, pectin, starch, and methylcellulose.

Since the CTL inducer of the present invention has a sufficient CTL inducing effect even without an adjuvant, it does not have to contain an adjuvant, but may contain an adjuvant. Examples of the adjuvant to be used include aluminum hydroxide gel, complete freund's adjuvant, incomplete freund's adjuvant, bordetella pertussis adjuvant, poly(I,C), and CpG-DNA.

The dose of the T cell epitope peptide in the preparation and number of doses of the preparation are different according to the kind of the T cell epitope peptide, the symptom, age and weight of the administration subject, and the dosage form, but the dose is usually 0.01 µg to 1 mg, preferably 0.1 µg to 500 µg, more preferably 1.0 µg to 100 µg, and it is preferable that such a dose is administered once in a few days to a few months. For example, in initial immunization (i.e. therapeutic or preventive administration), as for an adult patient, 1.0 µg to 500 µg of a peptide is administered, followed by boosting administration of 1.0 µg to 100 µg of a peptide in accordance with a boosting therapy over a few weeks to a few months, depending on the response and state of a patient by measurement of the specific CTL activity in the blood of the patient.

The CTL inducer of the present invention can be used as a vaccine for preventing or treating a viral disease, or a vaccine for preventing or treating various cancers.

The CTL inducer of the present invention can be administered to a human or an animal other than a human. Examples of the animal other than a human include livestock (e.g. cow, horse, pig, and chicken), pet animals (e.g. dog and cat), and experimental animals (mouse and rat).

The CTL inducer of the present invention can also be utilized in the following preventive or treatment methods.
(A) A method for preventing or treating a viral disease of a human, including administering, to a human, a virus-like particle composed of VP1 of simian virus 40 in which a T cell epitope peptide derived from a virus protein is inserted into a DE loop and/or an HI loop of the VP1.
(B) A method for preventing or treating a viral disease of an animal other than a human, including administering, to an animal other than a human, a virus-like particle composed of VP1 of simian virus 40 in which a T cell epitope peptide derived from a virus protein is inserted into a DE loop and/or an HI loop of the VP1.
(C) A method for preventing or treating a cancer of a human, including administering, to a human, a virus-like particle composed of VP1 of simian virus 40 in which a T cell epitope peptide derived from a protein specific to a cancer cell is inserted into a DE loop and/or an HI loop of the VP1.
(D) A method for preventing or treating a cancer of an animal other than a human, including administering, to an animal other than a human, a virus-like particle composed of VP1 of simian virus 40 in which a T cell epitope peptide derived from a protein specific to a cancer cell is inserted into a DE loop and/or an HI loop of the VP1.

### Examples

The present invention will be explained in more detail below by way of examples.

### [Example 1] Preparation of M1 CTL epitope-inserted SV40 VP1 gene

A CTL epitope sequence (GILGFVFTL) (this epitope sequence is an epitope sequence for HLA-A*0201 of human MHC class I) of an influenza virus particle internal protein M1 was inserted into SV40 VP1. Specifically, a DE loop region (137-138 amino acid region, classically, 4th Ala of a VP1 gene as amino acid No. 1) or an HI loop region (273-274 amino acid region) of VP1 was replaced with GILGFVFTL to prepare a M1 CTL epitope-inserted SV40 VP1 insertion mutant (M1-DE-VP1, M1-HI-VP1). The mutant was prepared by the Overhang PCR method by employing pFastBac 1-SV40 wild type VP1 encoding SV40 VP1 as a template. The following primers were used.

### Primers for preparing M1-DE-VP1

1^{st} round
   5'-SalI-Kozac-SV40 VP1
   AAAAGTCGACACCATGAAGATGGCCCCAACAAAAAG (SEQ ID No.: 40)
   3'-DE2 loop (M1)
   CGTGAACACAAAGCCCAAAATGCCGCCACCGCCATGAGTTTTTTGTGTCCCTGAATG
   (SEQ ID No.: 41)
   5'-DE2 loop (M1)
   CATTTTGGGCTTTGTGTTCACGTTGGGCGGCGGTGGTGCTGGAAAACCCATTCAAG
   (SEQ ID No.: 42)
   3'-KpnI-SV40 VP1
   AAAAGGTACCTCACTGCATTCTAGTTGTGGTTTG (SEQ ID No.: 43)
2^{nd} round
   5'-SalI-Kozac-SV40 VP1
   AAAAGTCGACACCATGAAGATGGCCCCAACAAAAAG (SEQ ID No.: 44)
   3'-KpnI-SV40 VP1
   AAAAGGTACCTCACTGCATTCTAGTTGTGGTTTG (SEQ ID No.: 45)

### Primers for preparing M1-HI-VP1

1^{st} round
   5'-SalI-Kozac-SV40 VP1
   AAAAGTCGACACCATGAAGATGGCCCCAACAAAAAG (SEQ ID No.: 46)
   3'-HI1 loop (M1)
   CGTGAACACAAAGCCCAAAATGCCGCCACCGCCGTTGGTAAACAGCCCACAAATG
   (SEQ ID No.: 47)
   5'-HI1 loop (M1)
   CATTTTGGGCTTTGTGTTCACGTTGGGCGGCGGTGGAACACAGCAGTGGAAGGG (SEQ
   ID No.: 48)
   3'-KpnI-SV40 VP1
   AAAAGGTACCTCACTGCATTCTAGTTGTGGTTTG (SEQ ID No.: 49)
2^{nd} round
   5'-SalI-Kozac-SV40 VP1
   AAAAGTCGACACCATGAAGATGGCCCCAACAAAAAG (SEQ ID No.: 50)
   3'-KpnI-SV40 VP1
   AAAAGGTACCTCACTGCATTCTAGTTGTGGTTTG (SEQ ID No.: 51)

Employing 10 ng of a template, each 50 pmol of primers were added to prepare 30 µL of a mixed solution having the concentrations of 2.5 units of a KOP polymerase, 0.2 mM dNTPs, 1 mM MgCl₂, 6 mM (NH₄)₂SO₄, 10 mM KCl, 120 mM Tris-HCl (pH 8.0), 0.1% Triton X-100, and 0.001% BSA.

In PCR, in both of 1st round and 2nd round, after incubation at 98°C for 60 seconds, the following cycle was repeated 25 times (98°C 15 seconds, 59°C 15 seconds, 74°C 30 seconds) and, finally, incubation was performed at 74°C for 1 minute and 30 seconds, and the procedure was migrated to 4°C.

PCR fragments prepared by the Overhang PCR were cut with restriction enzymes KpnI and SalI, and inserted between KpnI and SalI sites of a pFastBac 1 plasmid vector to obtain M1-DE-VP1 and M1-HI-VP1 plasmids.

### [Example 2] Preparation of baculovirus expressing M1-DE-VP1 and M1-HI-VP1

M1-DE-VP1 and M1-HI-VP1 plasmids were introduced into Escherichia coli DH10bac (Invitrogen) harboring a baculovirus genome to transform the Escherichia coli, and thereby a recombinant baculovirus genome with M1-DE-VP1 and M1-HI-VP1 incorporated therein was prepared. The recombinant baculovirus genome was transfected into a Sf-9 cell and, after 3 days, the supernatant was recovered to obtain a solution containing a recombinant baculovirus. The recombinant baculovirus titer was elevated by infecting a part of this solution with a Sf-9 cell again, to obtain a stock solution of the recombinant baculovirus.

### [Example 3] Preparation of M1-DE-VLP and M1-HI-VLP

With a recombinant baculovirus with M1-DE-VP1 and M1-HI-VP1 incorporated therein, 3 × 10⁷ Sf-9 cells were infected at M.O.I. (multiplicity of infection) = 0.05 to 0.2. Three days after infection, the Sf-9 cells were recovered and then washed with PBS (-), the cells were resuspended in 200 µl of a 50% Opti-prep™ solution (20 mM Tris-HCl (pH 7.9), 50% Opti-prep™), and ultrasonic-crushed to obtain a lysate solution (Fig. 1 left view, lanes 1 to 4, M1-DE-VP1 and right view, lanes 1 to 4, M1-HI-VP1). On the other hand, the cells were resuspended in 1 ml of a VP1 ultrasonicated solution (20 mM Tris-HCl (pH 7.9), 1% sodium deoxycholate), ultrasonic-crushed, and centrifuged at 15,000 rpm and 4°C for 5 minutes to fractionate into the supernatant and pellets, and the supernatant was removed. To the remaining pellets was added a 50% Opti-prep™ solution (20 mM Tris-HCl (pH 7.9), 50% Opti-prep™), and the mixture was resuspended by pipetting or ultrasonication to obtain a pellet solution (Fig. 1 left view, lanes 5 to 8, M1-DE-VP1 and right view, lanes 5 to 8, M1-HI-VP1).

A part of the prepared lysate and pellet solution was electrophoresed by SDS-PAGE, and stained by CBB staining (Fig. 1).

### [Example 4] Immunization with M1-DE-VLP and M1-HI-VLP

To the lysate solution (100 µl) and the pellet solution (100 µl) containing M1-DE-VLP and M1-HI-VLP was added 5 µg of a CpG-olygodeoxyncleotide5002 (TCCATGACGTTCTTGATGTT: SEQ ID No.: 52) adjuvant as an adjuvant, and this was immunized into an 8-week old transgenic mouse by a footpad, heel joint, intravenous, intraperitoneal, intramuscular or intranasal route. As the transgenic mouse, a transgenic mouse, a base of which is C57BL/6, and which expresses human β2m further fused with a chimera of HLA-A*0201 and H-2Db was used. In this mouse, since mouse β2m and H-2Db are knocked out, it is thought that mouse-derived MHC class I is not exposed to the cell surface.

### [Example 5] Preparation of lymphocyte from spleen and lung of immunized mouse

One week or two weeks after immunization, the spleen and lung were isolated from the mouse, and placed on a φ6 cm dish containing 5 ml of an RPMI-1640 medium, respectively. Using a pincette, the spleen was kneaded well in the medium, a solution containing lymphocytes which had been eluted into the medium was transferred to a 15 ml tube, the φ6 cm dish was washed again with 5 ml of the RPMI-1640 medium, and the supernatant was added to the 15 ml tube to a total amount of 10 ml. Immediately, while a tissue section accumulated on the bottom of the 15 ml tube was left, the supernatant was transferred again to a fresh 15 ml tube and centrifuged at room temperature and 1,200 rpm for 5 minutes to transfer lymphocytes to pellets. After the supernatant was removed and the pellets were loosened, in order to remove erythrocytes, 250 µl of a NH₄Cl-Tris solution was added and the materials were mixed, thereafter, 10 ml of an RPMI-1640 medium was added quickly, and the mixture was centrifuged at room temperature and 1,2000 rpm for 5 minutes to transfer lymphocytes to pellets. After the supernatant was removed and the pellets were loosened, 10 ml of an RPMI 1640 medium was added again, the mixture was transferred to a fresh 15 ml tube with a pipette so as not to absorb denatured erythrocytes, and this was centrifuged again at room temperature and 1,200 rpm for 5 minutes. After the supernatant was removed, pellets were loosened, suspended in 10 ml of an RPMI-1640 medium again, and the suspension was centrifuged at room temperature and 1,200 rpm for 5 minutes. The supernatant was removed, and the remainder was finally suspended in 2 ml of a 10% FCS-mixed RPMI-1640 medium. In order to count lymphocytes, 10 µl of the suspended solution was added to 490 µl of a 2% acetic acid solution, the cell number was counted with a Burker-Turk hemacytometer, and the cells were diluted with a 10% FCS-mixed RPMI-1640 medium or concentrated so that the cell number became 2 × 10⁷ cells/ml.

The isolated lung was transferred to a φ6 cm dish with no medium mixed therein, minced well with scissors, suspended in an RPMI-1640 medium with 10 ml of 50 units/ml Collagenase Type 1 and 10% FCS mixed therein, and incubated with a 5% CO₂ incubator at 37°C for 1 hour. After incubation, the suspension was passed through 100 µm of a cell strainer made of Nylon (BD Falcon) to recover the supernatant in a 50 ml tube, a tissue fragment remaining in the strainer was ground with a cell scraper, thereafter, the ground tissue fragment was further passed through 10 ml of a 10% FCS-mixed RPMI-1640 medium, and a total amount of 20 ml of a solution was recovered in a 50 ml tube. The recovered solution was centrifuged at room temperature and 1,200 rpm for 5 minutes. After the supernatant was removed and pellets were loosened, 10 ml of an RPMI-1640 medium was added, and the suspension was transferred to a 15 ml tube. The 15 ml tube was centrifuged at room temperature and 1,200 rpm for 5 minutes, the supernatant was removed and pellets were loosened, thereafter, 10 ml of an RPMI-1640 medium was further added, and the mixture was centrifuged at room temperature and 1,200 rpm for 5 minutes, thereby, cells were washed. After the supernatant was removed, pellets were loosened, and finally, 10 ml of a 10% FCS-mixed RPMI-1640 medium was added, the mixture was centrifuged at room temperature and 1,200 rpm for 5 minutes, the supernatant was removed and pellets were loosened and, thereafter, 250 µl of a 10% FCS-mixed RPMI-1640 medium was added.

### [Example 6] ICS analysis

In order to examine that a CTL which is reacted with a CTL epitope sequence (GILGFVFTL) of M1 to be induced is present in lymphocytes which were recovered from the spleen and lung by the aforementioned method, ICS analysis was performed. BD GolgiPlug™ (BD) which had been diluted 25-fold with a 10% FCS-mixed RPMI-1640 medium was added to a 96-well round bottom plate in an amount of 5 µl per well, and 100 µl of a 20 µM peptide (GILGFVFTL, Operon) of a CTL epitope of M1, which had been diluted with a 10% FCS-mixed RPMI-1640 medium, was further added thereto. As a negative control, 100 µl of a 10% RCS-mixed RPMI-1640 medium not containing a peptide was added.

To this well were added 100 µl of lymphocytes prepared from the spleen and lymphocytes prepared from the lung, which had been prepared as described above, and the mixture was incubated with a 5% CO₂ incubator at 37°C for 5 hours.

After incubation, the mixture was spun down at 4°C and 1,400 rpm to remove the supernatant and the cells were loosened with a vortex mixer, a FACS buffer (2% FCS, 0.1% sodium azide, 1 × PBS (-)) was added in an amount of 200 µl per well, the mixture was spun down again at 4°C and 1,400 rpm to remove the supernatant, the cells were loosened, and thereafter, 100 µl of Mouse BD Fc Block™ (BD Pharmingen) which had been diluted with a FACS buffer to 5 µg/ml was added, and the mixture was incubated at 4°C for 10 minutes.

After incubation, the mixture was spun down at 4°C and 1,400 rpm to remove the supernatant, and the cells were loosened, a FACS buffer was added in an amount of 200 µl per well, the mixture was spun down again at 4°C and 1,400 rpm to remove the supernatant, and the washing operation with a FACS buffer was performed again. To the loosened cells was added FICT Rat Anti-Mouse CD8a Clone: 53-6.7 (BD Pharmingen) which had been diluted with a FACS buffer to 10 µg/ml, in an amount of 50 µl per well, and this was incubated in a dark place at 4°C for 30 minutes.

After incubation, after an operation of washing with 200 µl of a FACS buffer was performed two times, to the loosened cells was added 100 µl of BD Cytofix/Cytoperm™ (BD Biosciences) per well, and this was incubated in a dark place at 4°C for 20 minutes. After incubation, after the same operation as the washing operation which had been performed with a FACS buffer was performed with 200 µl of 1 × BD Perm/Wash™ (BD Biosciences) two times, to the loosened cells was added 50 µl of PE anti-mouse IFN-γ Clone: XMG1.2 (BioLegend) which had been diluted with 1 × BD Perm/Wash^{TM} to 10 µg/ml, and the mixture was incubated in a dark place at 4°C for 30 minutes.

After incubation, after a washing operation with 200 µl of 1 × BD Perm/Wash™ was performed two times, to the loosened cells was added 100 µl of a FACS fixation buffer (1% formaldehyde, 1 × FACS buffer) per well, and this was incubated in a dark place at 4°C overnight.

After incubation, after 400 µl of a FACS buffer was added to a 5 ml polystyrene tube, and a sample fixed with 100 µl of a FACS fixation buffer was added thereto, dot plot analysis was performed with FACScan (BD). For the analysis, the software of Cell Quest (BD) was used. The result of two-dimensional analysis of ICS is shown in Fig. 2 and Fig. 3.

As shown in Fig. 2, the ratio of the number of CD8 positive, intracellular IFN-γ positive cells was increased by incubation of a CTL epitope peptide in all the measurement results. Further, the result that the ratio of the number of CD8 positive, intracellular IFN-γ positive cells is increased when CpG is not added was also obtained. From them, it was understood that there is a possibility that M1-DE-VLP and M1-HI-VLP can induce a CTL even without an adjuvant.

As shown in Fig. 3, when M1-DE-VLP was administered intramuscularly, and when M1-HI-VLP was administered intraperitoneally, increase in the number of CD8 positive, intracellular IFN-γ positive cells by incubation of a CTL epitope peptide was remarkable.

### [Example 7] CD107-ICS analysis

In order to examine that a CTL having the cytotoxic activity, which is reacted with a CTL epitope sequence (GILGFVFTL) of M1 to be induced, is present in lymphocytes which were recovered from the spleen by the method of Example 5, CD107-ICS analysis was performed. To a 96-well round bottom plate were added BD GolgiPlug™ (BD) which had been diluted 25-fold with a 10% FCS-mixed RPMI-1640 medium in an amount of 5 µl per well, BD Golgi Stop™ (BD) which had been diluted 25-fold with a 10% FCS-mixed RPMI-1640 medium in an amount of 5 µl per well, and 0.8 µg of FITC Rat Anti-Mouse CD107a Clone: 1D4B (BD Pharmingen), and 100 µl of a 20 µM peptide (GILGFVFTL, Operon) of a CTL epitope of M1 which had been diluted with a 10% FCS-mixed RPMI-1640 medium was further added thereto. As a negative control, 100 µl of a 10% RCS-mixed RPMI-1640 medium not containing a peptide was added.

After 100 µl of lymphocytes prepared from the spleen, which had been prepared in Example 5, were added to this well, the mixture was incubated with a 5% CO₂ incubator in a dark place at 37°C for 6 hours.

After incubation, the mixture was spun down at 4°C and 1,400 rpm to remove the supernatant and the cells were loosened with a vortex mixer, a FACS buffer (2% FCS, 0.1% sodium azide, 1 × PBS (-)) was added in an amount of 200 µl per well, the mixture was spun down again at 4°C and 1,400 rpm to remove the supernatant, the cells were loosened, and thereafter, 100 µl of Mouse BD Fc Block™ (BD Pharmingen) which had been diluted with a FACS buffer to 5 µg/ml was added, and the mixture was incubated at 4°C for 10 minutes.

After incubation, the mixture was spun down at 4°C and 1,400 rpm to remove the supernatant, and the cells were loosened, a FACS buffer was added in an amount of 200 µl per well, the mixture was spun down again at 4°C and 1,400 rpm to remove the supernatant, and the washing operation with a FACS buffer was performed again. To the loosened cells was added PE/Cy5 Rat Anti-Mouse CD8a Clone: 53-6.7 (BD Pharmingen) which had been diluted with a FACS buffer to 10 µg/ml, in an amount of 50 µl per well, and this was incubated in a dark place at 4°C for 30 minutes.

After incubation, after an operation of washing with 200 µl of a FACS buffer was performed two times, to the loosened cells was added 100 µl of BD Cytofix/Cytoperm™ (BD Biosciences) per well, and this was incubated in a dark place at 4°C for 20 minutes. After incubation, after the same operation as the washing operation which had been performed with a FACS buffer was performed with 200 µl of 1 × BD Perm/Wash™ (BD Biosciences) two times, to the loosened cells was added 50 µl of PE anti-mouse IFN-γ Clone: XMG1.2 (BioLegend) which had been diluted with 1 × BD Perm/Wash™ to 10 µg/ml, and the mixture was incubated in a dark place at 4°C for 30 minutes.

After incubation, after a washing operation with 200 µl of 1 × BD Perm/Wash™ was performed two times, to the loosened cells was added 100 µl of a FACS fixation buffer (1% formaldehyde, 1 × FACS buffer) per well, and this was incubated in a dark place at 4°C overnight.

After incubation, after 400 µl of a FACS buffer was added to a 5 ml polystyrene tube, and a sample fixed with 100 µl of a FACS fixation buffer was added thereto, dot plot analysis was performed with FACScan (BD). For the analysis, the software of Cell Quest (BD) was used. The result of two-dimensional analysis of CD107-ICS is shown in Fig. 4.

As shown in Fig. 4, it was understood that when the ratio of the number of CD8 positive, intracellular IFN-γ positive cells is increased by incubation of a CTL epitope peptide, the ratio of the number of CD8 positive, CD107 positive, intracellular IFN-γ positive cells is also increased in all the measurement results. Since a CD8 positive, CD107 positive, intracellular IFN-γ positive cell has the cytotoxic activity, it was understood that there is a possibility that immunization with M1-DE-VLP and M1-HI-VLP can induce a CTL having cytotoxicity.

### [Example 8] ⁵¹Chrome release analysis

In order to show that the CD8 positive, CD107 positive, intracellular IFN-γ positive cell shown in Example 7 damages a cell, ⁵¹Chrome release analysis was performed. Lymphocytes of the spleen were prepared from a non-immunized mouse by the same method as that of Example 5. To 2.4 × 10⁸ cells of spleen lymphocytes of a non-immunized mouse in 2 ml of a 10% FCS-mixed RPMI-1640 medium was added 4 µl of a 10 mM peptide (GILGFVFTL, Operon) of a CTL epitope of M1, and the mixture was incubated with a 5% CO₂ incubator at 37°C for 2 hours in a 15 ml tube. After incubation, 20 Gy (gray) X-ray was radiated. After the radiation, the mixture was centrifuged at room temperature and 1,2000 rpm for 5 minutes to transfer lymphocytes to pellets. After the supernatant was removed and pellets were loosened, 2 ml of a 10% FCS-mixed RPMI-1640 medium was added, and in order to count lymphocytes, 10 µl of a lymphocyte solution was added to 490 µl of a 2% acetic acid solution, the cell number was calculated with a Burker-Turk hemacytometer, and this was diluted with a 10% FCS-mixed RPMI-1640 medium to 5 × 10⁶ cells/ml.

Simultaneously, about 50 µg of M1-DE-VLP was immunized by intramuscular injection, and about 50 µg of M1-HI-VLP was immunized by intraperitoneal injection and, one week after immunization, the spleen was isolated from a mouse, and placed on a φ6 cm dish containing 5 ml of an RPMI-1640 medium. Using a pincette, the spleen was kneaded well in the medium, a solution containing lymphocytes which had been eluted into the medium was transferred to a 15 ml tube, the φ6 cm dish was washed again with 5 ml of the RPMI-1640 medium, and the supernatant was added to the 15 ml tube to a total amount of 10 ml. Since the blood was not removed from the immunized lymphocytes, immediately, while a tissue section accumulated on the bottom of the 15 ml tube was left, the supernatant was transferred again to a fresh 15 ml tube and centrifuged at room temperature and 1,200 rpm for 5 minutes to transfer lymphocytes to pellets. After the supernatant was removed and pellets were loosened, 2 ml of a 10% FCS-mixed RPMI-1640 medium was added, and in order to count lymphocytes, 10 µl of a lymphocyte solution was added to 490 µl of a 2% acetic acid solution, the cell number was calculated with a Burker-Turk hemacytometer, and this was diluted with a 10% FCS-mixed RPMI-1640 medium to 5 × 10⁶ cells/ml.

Into a 48-well plate were mixed 500 µl of 5 × 10⁶ cells/ml of lymphocytes which had been incubated with a peptide of a CTL epitope of M1 and irradiated with an X-ray, and which had not been immunized, and 500 µl of 5 × 10⁶ cells/ml of immunized lymphocytes, per well. Twenty four wells per sample were prepared. After mixing, the mixture was incubated with a 5% CO₂ incubator at 37°C for 7 days.

After incubation for 7 days, lymphocytes in 24 wells were collected in one 50 ml tube, and centrifuged at room temperature and 1,200 rpm for 5 minutes to transfer lymphocytes to pellets. After the supernatant was removed and pellets were loosened, 2 ml of a 10% FCS-mixed RPMI-1640 medium was added, and in order to count lymphocytes, 20 µl of a lymphocyte solution was added to 20 µl of a 0.4% trypan blue solution (Gibco), the cell number was calculated with a Burker-Turk hemacytometer, and the cells were diluted with a 10% FCS-mixed RPMI-1640 medium or concentrated, so that the cell number became 7.5 × 10⁶ cells/ml. The prepared lymphocytes were used as effector cells.

Simultaneously, each 1 ml of 1 × 10⁶ cells/ml of RMA-HHD cultured cells were dispensed into two 15 ml tubes, 5 µl of a 10 mM peptide (GILGFVFTL, Operon) of a CTL epitope of M1 was added to one of the tubes, and this was incubated with a 5% CO₂ incubator at 37°C for 2 hours. No peptide was added to the other tube. After incubation, the tube was centrifuged at room temperature and 1,000 rpm for 5 minutes to transfer cells to pellets. After the supernatant was removed and pellets were loosened, 100 µCi (microsievert) of a Na₂⁵¹CrO₄ solution wad added, and this was incubated with a 5% CO₂ incubator at 37°C for 30 minutes. After incubation, the tube was centrifuged at room temperature and 1,000 rpm for 5 minutes to transfer cells to pellets. After the supernatant was removed and pellets were loosened, 1 ml of a 10% FCS-mixed RPMI-1640 medium was added for a washing operation, this was centrifuged at room temperature and 1,200 rpm for 5 minutes to transfer lymphocytes to pellets, and the supernatant was removed. This washing operation was repeated five times. Finally, after the supernatant was removed, 1 ml of a 10% FCS-mixed RPMI-1640 medium was added. Into a fresh 15 ml tube were mixed 500 µl of this cell solution and 9.5 ml of a 10% FCS-mixed RPMI 1640 medium to dilute the cells 20-fold. This cell solution was used as target cells.

Into a 96-well plate was dispensed 100 µl of target cells (5 × 10³ cells), 100 µl of the effector cells (7.5 × 10⁵ cells) were added thereto so that the ratio of effector cell: target cell became 150: 1, 20 µl of the effector cells (1.5 × 10⁵ cells) and 80 µl of a 10% FCS-mixed RPMI-1640 medium were added thereto so that the ratio of effector cell: target cell became 30: 1, and 10 µl of the effector cells (7.5 × 10⁴ cells) and 90 µl of a 10% FCS-mixed RPMI-1640 medium were added thereto so that the ratio of effector cell: target cell became 15: 1. In addition, 100 µl of a 5% Triton-X 100 solution as a positive control, and 100 µl of a 10% FCS-mixed RPMI-1640 medium as a negative control were added. After mixing, the mixture was incubated with a 5% CO₂ incubator at 37°C for 4 hours.

After incubation, the supernatant was recovered using Supernatant collection system (Molecular Devices), a gamma ray of released ⁵¹Cr was counted and analyzed with AUTO WELL GAMMA SYSTEM (ARC-380CL, Aloka) and ALOKA RIA Program (ARCAS ver. 3.11, Aloka). The result of ⁵¹Chrome release analysis is shown in Fig. 5.

As shown in Fig. 5, since lymphocytes which were prepared by immunization with M1-DE-VLP and M1-HI-VLP induce release of ⁵¹Cr by selectively damaging a cell (M1: 58-66 of Fig. 5) presenting a peptide (GILGFVFTL) of a CTL epitope of M1, it was understood that immunization with M1-DE-VLP and M1-HI-VLP can induce a CTL which selectively damages a cell presenting a peptide (GILGFVFTL) of a CTL epitope of M1.

### [Example 9] In vivo CTL analysis

In order to analyze that the CTL which selectively damages a cell presenting a peptide (GILGFVFTL) of a CTL epitope of M1, shown in Example 8, selectively damages a cell actually presenting a peptide (GILGFVFTL) of a CTL epitope of M1 in an animal body, In vivo CTR analysis was performed. About 50 µg of M1-DE-VLP was immunized by intramuscular injection, and about 50 µg of M1-HI-VLP was immunized by intraperitoneal injection.

One week after immunization, the spleen was isolated from three non-immunized mice, and placed on a φ6 cm dish containing 5 ml of an RPMI-1640 medium. Using a pincette, the spleen was kneaded well in the medium, a solution containing lymphocytes which had been eluted into the medium was transferred to a 15 ml tube, the φ6 cm dish was washed again with 5 ml of the RPMI-1640 medium, and the supernatant was added to the 15 ml tube to a total amount of 10 ml. Since blood was not removed from non-immunized lymphocytes, immediately, while a tissue section accumulated on a bottom of the 15 ml tube was left, the supernatant was transferred again to a fresh 15 ml tube and centrifuged at room temperature and 1,200 rpm for 5 minutes to transfer lymphocytes to pellets. After the supernatant was removed and pellets were loosened, 2 ml of a 10% FCS-mixed RPMI-1640 medium was added. Into two 15 ml tubes were dispensed each 1 ml of prepared non-immunized lymphocytes, 1 µl of a 10 mM peptide (GILGFVFTL, Operon) of a CTL epitope of M1 was added to one of the tubes, and this was incubated with a 5% CO₂ incubator at 37°C for 1 hour. After incubation, the mixture was centrifuged at room temperature and 1,2000 rpm for 5 minutes to transfer lymphocytes to pellets. After the supernatant was removed and pellets were loosened, 10 ml of an RPMI-1640 medium was added again, the mixture was centrifuged at room temperature and 1,2000 rpm to transfer lymphocytes to pellets. After the supernatant was removed and pellets were loosened, 20 ml of a 0.1% BSA-mixed PBS (-) solution was added, 10 µl of a 5 mM carboxyfluorescein diacetate succinimidyl ester (CFSE) (Molecular Probes) was added to a lymphocyte solution which had been incubated with a peptide (GILGFVFTL) of a CTL epitope, 1 µl of 5 mM CFSE was added to a lymphocyte solution which had not been incubated with a peptide, the materials were mixed well and, thereafter, the mixture was shaking-cultured with a water bath at 37°C for 10 minutes. After shaking, the mixture was centrifuged at room temperature and 1,200 rpm for 5 minutes to transfer lymphocytes to pellets. After the supernatant was removed and pellets were loosened, 10 ml of a 10% FCS-mixed RPMI-1640 medium was added, and the mixture was centrifuged at room temperature and 1,200 rpm for 5 minutes to transfer lymphocytes to pellets. After the supernatant was removed and pellets were loosened, 2 ml of a 10% FCS-mixed RPMI-1640 medium was added, and in order to count lymphocytes, 10 µl of a lymphocyte solution was added to 490 µl of a 2% acetic acid solution, the cell number was calculated with a Burker-Turk hemacytometer, and the cells were diluted with a 10% FCS-mixed RPMI-1640 medium or concentrated, so that the cell number became 5 × 10⁷ cells/ml. The same amount of 5 × 10⁷ cells/ml of lymphocytes which had not been incubated with a peptide labeled with CFSE and 5 × 10⁷ cells/ml of lymphocytes which had been incubated therewith were mixed to obtain a CFSE-labeled lymphocyte solution.

Each of a non-immunized mouse, and a mouse after one week from immunization with about 50 µg of M1-DE-VLP by intramuscular injection and about 50 µg of M1-HI-VLP by intraperitoneal injection was intravenously injected with 200 µl of the above-prepared CFSE-labeled lymphocyte solution, and left for one day.

After one day had passed, the spleen was isolated from the mouse, and placed on a φ6 cm dish containing a 5 ml of an RPMI-1640 medium. Using a pincette, the spleen was kneaded well in the medium, a solution containing lymphocytes which had been eluted into the medium was transferred to a 15 ml tube, the φ6 cm dish was washed again with 5 ml of the RPMI-1640 medium, and the supernatant was added to the 15 ml tube to a total amount of 10 ml. Since blood was not removed from lymphocytes, immediately, while a tissue section accumulated on the bottom of the 15 ml tube was left, the supernatant was transferred again to a fresh 15 ml tube and centrifuged at room temperature and 1,200 rpm for 5 minutes to transfer lymphocytes to pellets. After the supernatant was removed and pellets were loosened, 1 ml of a FACS fixation buffer (1% formaldehyde, 1 × FACS buffer) was added. To a 5 ml polystyrene tube was added 500 µl of a FACS buffer, 200 µl of a lymphocyte solution which had been fixed with the FACS fixation buffer was added thereto, and histogram analysis was performed with FACScan (BD). For the analysis, the software of Cell Quest (BD) was used. The result of In vivo CTL analysis is shown in Fig. 6.

As shown in Fig. 6, when lymphocytes were prepared from a non-immunized mouse, the cell number of lymphocytes with no CTL epitope sequence mixed therein (shown at the position of low in the graph) and the cell number of lymphocytes with a CTL epitope sequence mixed therein (shown at the position of high in the graph) were approximately the same. On the other hand, when lymphocytes were prepared from a mouse immunized with M1-DE-VLP or M1-HI-VLP, the cell number of lymphocytes with a CTL epitope sequence mixed therein (shown at the position of high in the graph) was smaller as compared with the cell number of lymphocytes with no CTL epitope sequence mixed therein (shown at the position of low in the graph). From this result, it was understood that a CTL which is induced by immunization with M1-DE-VLP or M1-HI-VLP selectively damages a cell actually presenting a peptide of a CTL epitope of M1 in an animal body.

### [Example 10] Analysis of maturation of lymphocytes

According to the same method as that of Example 5, lymphocytes, from which erythrocytes had been removed, were purified from the spleen of a C57BL/6 mouse, and diluted with a 10% FCS-mixed RPMI-1640 medium to 1 × 10⁷ cells/ml. To a 96-well plate was added a) 5 µl of a 50% Opti-prep™ solution with no substance added thereto (20 mM Tris-HCl (pH 7.9), 50% Opti-prep™), b) 5 µl of a M1-DE-VLP solution prepared in Example 3, or c) 5 µl of a M1-HI-VLP solution, 50 µl of the above-prepared lymphocytes were added thereto, and a 10% FCS-mixed RPMI-1640 medium was added thereto so that the total amount per well became 200 µl. After mixing by pipetting, this plate was incubated with a 5% CO₂ incubator at 37°C for 6 hours or 24 hours.

After incubation, after the mixture was spun down at 4°C and 1,400 rpm to remove the supernatant and the cells were loosened with a vortex mixer, 200 µl of a FACS buffer (2% FCS, 0.1% sodium azide, 1 × PBS (-)) was added per well, the mixture was spun down again at 4°C and 1,400 rpm to remove the supernatant, and the cells were loosened, thereafter, 100 µl of FITC American Hamster Anti-Mouse CD80 Clone: 16-10A1 (BD Pharmingen), FITC Rat Anti-Mouse CD86 Clone: GL-1 (BD Pharmingen), or FITC American Hamster Anti-Mouse CD40 Clone: HM40-3 (BD Pharmingen), which had been diluted with a FACS buffer to 10 µg/ml, was added, and this was incubated in a dark place at 4°C for 30 minutes.

After incubation, after an operation of washing with 200 µl of a FACS buffer was performed two times, to loosened cells was added 100 µl of a FACS fixation buffer (1% formaldehyde, 1 × FACS buffer) per well, and the mixture was incubated in a dark place at 4°C overnight.

After incubation, 400 µl of a FACS buffer was added to a 5 ml polystyrene tube, a sample which had been fixed with 100 µl of a FACS fixation buffer was added thereto, and histogram analysis was performed with FACScan (BD). For the analysis, the software of Cell Quest (BD) was used. The result of histogram is shown in Fig. 7.

As shown in Fig. 7, while a 50% Opti-prep™ solution did not induce expression of maturation markers of CD80, CD86 and CD40, M1-DE-VLP- and M1-HI-VLP-mixed solutions induced expression of maturation markers of CD86 and CD40. Since expression of CD86 is important for activating a cytotoxic T lymphocyte, it was understood that M1-DE-VLP and M1-HI-VLP have the action of activating a cytotoxic T lymphocyte.

### [Example 11] Virus challenge experiment

In order to analyze the influence of a CTL which selectively damages a cell presenting a peptide (GILGFVFTL) of a CTL epitope of M1 on influenza virus infection, a virus challenge experiment was performed. About 50 µg of M1-DE-VLP was immunized into a HLA-A2 transgenic mouse (HHD mouse) by intramuscular injection, and about 50 µg of M1-HI-VLP was immunized into the mouse by intraperitoneal injection.

One week after immunization, influenza viruses (A/H1N1/PR8 and A/H3N2/Aichi) were diluted with PBS (-) so that the tissue culture influenza virus infectious dose (TCID₅₀) became 100 TCID₅₀/20 µl, and an immunized mouse was anesthetized and nasally infected with 20 µl of this diluted solution.

Four days after infection, the mouse lung was taken out, 1 ml of PBS (-) was added, the mouse lung was ground with a tissue homogenizer (glass homogenizer, warty, 10 ml, 812-770-02, Ikemoto Scientific Technology Co., Ltd.) until the tissue became invisible, and the solution was transferred to a 15 ml tube. The tube was centrifuged with a centrifuge at 4°C and 2000 rpm for 5 minutes, and the supernatant was transferred to a 2 ml tube equipped with a screw cap. In order to calculate the amount of influenza virus from hemagglutination by adding erythrocytes of chicken, 100 µl of the supernatant from grinding of the mouse lung was added to a lane A of a round bottom 96-well plate, 90 µl of a 5% FCS-mixed DMEM medium wad added to lanes B to G and, as a negative control, 100 µl of a 5% FCS-mixed DMEM medium was added to a lane H. A repeating operation of transferring 10 µl of the liquid from the lane A to the lane B, suspending it by pipetting and, thereafter, transferring 10 µl of the liquid from the lane B to the lane C, and suspending it was performed up to the lane G, to prepare a 10-fold dilution series of the supernatant of lung infected with an influenza virus. In order to calculate the average, five series were prepared per one sample. After each 100 µl of 2.5 × 10⁵ cells/ml of MDCK cells were added to all of the series, the mixture was incubated with a 5% CO₂ incubator at 35°C for 24 hours. After incubation, after all of the culturing supernatant was removed with a pipette and 200 µl of a 0.0002% trypsin-mixed (2 µl/ml trypsin) DMEM medium was added, the mixture was further incubated with a 5% CO₂ incubator at 35°C for 3 days. After incubation, 50 µl of 0.8% chicken blood (chicken stored blood, 0109-1, Nippon Bio-Test Laboratories Inc.) was added, and this was allowed to stand at 4°C for 1 hour. From the distribution of hemagglutination, the tissue culture influenza virus infectious dose (TCID₅₀) was calculated.

As shown in Fig. 8a, since the influenza virus titer in the mouse lung immunized with M1-DE-VLP and M1-HI-VLP is lower about 10 times as compared with that of a non-immunized mouse, it was suggested that M1-DE-VLP and M1-HI-VLP immunization has an effect of inhibiting proliferation of an influenza virus in the lung.

Further, from the influenza virus (A/H1N1/PR8) infection, the weight of mouse was weighed every day for 2 weeks, and the change in weight was recorded regarding the weight before infection as 100%. As shown in Fig. 8b, while a mouse immunized with M1-DE-VLP and M1-HI-VLP substantially maintained the original weight, a remarkable decrease in weight was observed in a non-immunized mouse. Therefore, it was suggested that immunization with M1-DE-VLP and M1-HI-VLP has an effect also on inhibition of decrease in weight by influenza virus infection.

### [Example 12] Immunization with VLP with cancer CTL epitope inserted therein

According to the same methods as those of Examples 1 to 3, a VLP in which a CTL epitope sequence (RMFPNAPYL) for HLA-A2 of WT1 known as a cancer antigen is inserted in place of a CTL epitope sequence (GILGFVFTL) of M1 was prepared (WT1WT-DE-VLP, WT1WT-HI-VLP) (Fig. 9-a). According to the same methods as those of Examples 4 to 6, a HLA-A2 transgenic mouse was immunized to prepare lymphocytes, and ICS analysis on a CTL epitope sequence of WT1 was performed (Fig. 9-b). Further, according to the same method as that of Example 8, ⁵¹Chrome release analysis on a CTL epitope sequence of WT1 was performed.

It was revealed that, as shown in Fig. 9, by preparing a VLP with a CTL epitope sequence of WT1 inserted therein similarly to a VLP with a CTL epitope sequence of M1 inserted therein (Fig. 9-a) and performing immunization, a CTL for a CTL epitope sequence (RMFPNAPYL) of WT1 is induced (Fig. 9-b), and the induced CTL for a CTL epitope of WT1 selectively damages a cell presenting a CTL epitope sequence of WT1 (Fig. 9-c). From this result, it was demonstrated that a VLP is a useful carrier which can induce a CTL for a variety of CTL epitope sequences.

### [Example 13] ICS analysis of CTL induction by M1 CTL peptide-suspended IFA emulsion

According to the same methods as those of Examples 3 to 6, ICS analysis of M1-DE-VLP (Fig. 10, right upper view) and M1-HI-VLP (Fig. 10, right lower view) was performed. Immunization was performed by injecting 100 µl of a pellet solution of M1-DE-VLP and M1-HI-VLP, which had been prepared as in Example 4, into the inguinal area subcutaneously (s.c.).

On the other hand, an IFA emulsion in which a peptide (GILGFVFTL) of a CTL epitope of M1 is suspended was prepared by diluting 50 µl of a GILGFVFTL peptide (Operon), which had been dissolved in a DMSO solvent having the concentration of 20 µg/µl, with 450 µl of PBS (-), and kneading the diluted peptide with 500 µl of an incomplete freund's adjuvant (IFA) (Sigma) by the Two-syringe method (GP syringe connector, NIPRO) (Inject 2 ml, B. BRAUN). Immunization was performed by injecting 100 µl of the prepared emulsion into the inguinal area subcutaneously (s.c.). A mouse was subjected to first immunization (Fig. 10, left upper view) and second immunization (Fig. 10, left lower view) one week after first immunization and, after one week, the mouse was used to perform ICS analysis according to the same methods as those of Examples 5 to 6.

As shown in Fig. 10, the ratio of CD8 positive, intracellular IFN-γ positive CTL which is induced by incubation with a M1 CTL peptide was higher in spleen lymphocytes of a mouse which was immunized with M1-DE-VLP and M1-HI-VLP once than in spleen lymphocytes of a mouse which was subjected to first immunization and second immunization with a M1 CTL peptide-suspended IFA emulsion. From this result, it was suggested that the efficiency of inducing CD8 positive, intracellular IFN-γ positive CTL is better in the cytotoxic T lymphocyte inducer of the present invention constructed using a VLP than in the inducer constructed using IFA known as a classical adjuvant.

### [Example 14] ELISA analysis of secretion amount of IL-12 of spleen lymphocytes

According to the same method as that of Example 5, lymphocytes from which erythrocytes had been removed were purified from the spleen of a HLA-A2 transgenic mouse, and diluted with a 10% FCS-mixed RPMI-1640 medium to 1 × 10⁷ cells/ml. To a 96-well plate was added a) 5 µl of a 50% Opti-prep™ solution with no substance added thereto (20 mM Tris-HCl (pH 7.9), 50% Opti-prep™), b) 5 µl of a highly purified wild type SV40 VP1-VLP (500 ng/µl), c) 5 µl of a M1-DE-VLP solution prepared in Example 3, or d) 5 µl of a M1-HI-VLP solution, 50 µl of the above-prepared lymphocytes were added thereto, and a 10% FCS-mixed RPMI-1640 medium was added thereto so that the total amount per well became 200 µl. Alternatively, 150 µl of LPS (Sigma) which had been diluted with a 10% FCS-mixed RPMI-1640 medium to 100 ng/µl was added to 50 µl of the above-purified lymphocytes. After mixing by pipetting, this plate was incubated with a 5% CO₂ incubator at 37°C for 6 hours or 24 hours. After incubation, the mixture was spun down at 4°C and 1,400 rpm, and the supernatant was recovered in an Eppendorf tube, the remainder was spun down at 4°C and 15,000 rpm, and the supernatant was recovered into a fresh Eppendorf tube. According to the protocol of the kit, 50 µl of the recovered culturing supernatant was analyzed by an enzyme-linked immunosorbent assay (ELISA) (Mouse Total IL-12 ELISA Kit, Thermo SCIENTIFIC) of IL-12, and the concentration of IL-12 in the culturing supernatant was calculated (Fig. 11).

As shown in Fig. 11, when lymphocytes of the mouse and a VLP in which an exogenous CTL epitope of M1 is inserted into a DE loop or an HI loop of a VLP (M1-DE-VLP, M1-HI-VLP) were incubated, the concentration of IL-12 in the culturing supernatant was increased about 20 times as compared with wild type SV40 VP1 VLP. Therefore, it was shown that, by inserting an exogenous epitope, the activity as an adjuvant is potentiated and an effect as a cytotoxic T lymphocyte inducer is increased compared with a wild type VLP.

### Industrial Applicability

Since the present invention is useful as a preventive or a therapeutic for a viral disease and a cancer, it can be utilized in an industrial field such as pharmacy.

The present specification includes the contents described in the specification and/or the drawings of Japanese patent applications (Patent application Nos. 2011-107874 and 2011-191208) which are the basis of priority claim of the present application. Further, all publications, patents and patent applications cited in the present specification are incorporated herein by reference, as they are.

## Claims

1. A cytotoxic T lymphocyte inducer comprising a virus-like particle composed of VP1 of simian virus 40, wherein a T cell epitope peptide is inserted into a DE loop and/or an HI loop of the VP1.

2. The cytotoxic T lymphocyte inducer according to claim 1, wherein the T cell epitope peptide is a peptide derived from a virus protein.

3. The cytotoxic T lymphocyte inducer according to claim 1, wherein the T cell epitope peptide is a peptide derived from HA, NA, M1, M2, NP, NS1, NS2, PA, PB1, PB2 or PB1-F2 of an influenza virus.

4. The cytotoxic T lymphocyte inducer according to claim 1, wherein the T cell epitope peptide is a peptide derived from a protein specific to a cancer cell.

5. The cytotoxic T lymphocyte inducer according to claim 1, wherein the T cell epitope peptide is a peptide derived from Melan-A/MART-1, gp100, MAGE-A3, MAGE-A10, CEA, HER2/new, NY-E50-1, WT-1 or hTERT.

6. The cytotoxic T lymphocyte inducer according to claim 1, wherein the T cell epitope peptide is a peptide consisting of an amino acid sequence described in any of SEQ ID Nos.: 1 to 39.

7. A method for preventing or treating a viral disease of an animal other than a human, comprising administering, to an animal other than a human, a virus-like particle composed of VP1 of simian virus 40 in which a T cell epitope peptide derived from a virus protein is inserted into a DE loop and/or an HI loop of the VP1.

8. A method for preventing or treating a cancer of an animal other than a human, comprising administering, to an animal other than a human, a virus-like particle composed of VP1 of simian virus 40 in which a T cell epitope peptide derived from a protein specific to a cancer cell is inserted into a DE loop and/or an HI loop of the VP1.

9. A method for preventing or treating a viral disease of a human, comprising administering, to a human, a virus-like particle composed of VP1 of simian virus 40 in which a T cell epitope peptide derived from a virus protein is inserted into a DE loop and/or an HI loop of the VP1.

10. A method for preventing or treating a cancer of a human, comprising administering, to a human, a virus-like particle composed of VP1 of simian virus 40 in which a T cell epitope peptide derived from a protein specific to a cancer cell is inserted into a DE loop and/or an HI loop of the VP1.
